# EUROPEAN PATENT APPLICATION

(11) **EP 2 238 979 A1**
(43) Date of publication of application: **13.10.2010**
(21) Application number: 09005047.7
(22) Date of filing: 06.04.2009
(51) Int. Cl.: A61K 31/4985, A61K 9/14, A61K 9/20, A61K 47/02

(54) **Active pharmaceutical ingredient adsorbed on solid support**

(71) Applicant: LEK Pharmaceuticals d.d., 1526 Ljubljana (SI)
(72) Inventor: The designation of the inventor has not yet been filed

(57) **Abstract**

Dosage forms comprising tadalafil adsorbed on a particulate carrier. Furthermore it is disclosed an adsorbate comprising tadalafil adsorbed on a particulate carrier. The present invention also refers to a process for the preparation of the adsorbate comprising the steps of (a) providing an API being practically insoluble in water; (b) providing solid support carrier particles; (c) placing the API and the carrier particles into a predetermined solvent which, allows adsorption of the API on the surface of carrier particles but gives substantially no precipitate, and (d) removing the solvent to form an API-carrier adsorbate.
Furthermore the invention relates to a process for the preparation of the dosage form, as well as to the use of the adsorbate for the preparation of the dosage form. Moreover it relates to the dosage form for use in the treatment of erectile dysfunction, hypercholesterolemia, human immunodeficiency virus (HIV) infections and/or Acquired Immune Deficiency Syndrome (AIDS).

## Description

### Field of the invention

The present invention belongs to the field of pharmaceutical industry and relates to dosage forms comprising active pharmaceutical ingredients (APIs) that are practically insoluble in water, such as tadalafil, on a carrier. Furthermore it relates to an adsorbate comprising tadalafil on a carrier and a process for the preparation of the adsorbate and the dosage form, as well as to the use of the adsorbate for the preparation of the dosage form. Moreover it relates to the dosage form for use in the treatment of diseases such as erectile dysfunction, hypercholesterolemia, human immunodeficiency virus (HIV) infections or Acquired Immune Deficiency Syndrome (AIDS). The present invention further relates to the preparation of an adsorbate which can be generally applied to an API having a low solubility in water and which is particularly suitable for preparing an adsorbate comprising tadalafil on a carrier.

### Description of the background art

The manufacturing of dosage forms containing a practically insoluble active pharmaceutical ingredient (API) and having excellent dissolution characteristics is a great challenge in the pharmaceutical industry. An API can for instance be an API selected from the group consisting of proteins, peptides, nucleotides, anti-obesity drugs, nutraceuticals, corticosteroids, elastase inhibitors, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoreceptor blocking agents, blood products and substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and bisphosphonates, prostaglandins, radio-pharmaceuticals, sex hormones, anti-allergic agents, stimulants and anorectics, sympathomimetics, thyroid agents, vasodilators, and xanthines. APIs being practically insoluble in water are for instance tadalafil, fenofibrate, cholecalciferol (vitamin D), simvastatin, NSAIDs (non-steroidal anti-inflammatory drugs), such as naproxen and ibuprofen, megestrol acetate, ritonavir (HIV protease inhibitor), or ciclosporin. Suitable dosage forms should release the complete amount of API with a desired dissolution rate and should also have the desired stability and other properties which are important with respect to dosage forms.

As mentioned above, one example for such an practically insoluble API is (6R,12aR)-6-(1,3-Benzodioxol-5-yl)-2,3,6,7,12,12a-hexahydro-2-methyl-pyrazino[1',2':1,6]pyrido[3,4-b]indole-1,4-dione (tadalafil). Tadalafil is a potent and selective inhibitor of the cyclic guanosine monophosphate (cGMP)-specific phosphodiesterase enzyme PDE5 having the formula

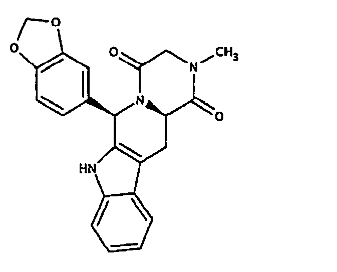

Like most of the poorly soluble or practically insoluble APIs, respectively, tadalafil is present as a crystalline solid that is practically insoluble in water. However, it should be noted that there might be other practically insoluble APIs that are present in an amorphous state. The inhibition of PDE5 increases the amount of cGMP, resulting in smooth muscle relaxation and increased blood flow. Tadalafil is therefore currently used for instance in the treatment of pulmonary arterial hypertension, or sexual dysfunctions, such as male erectile dysfunction.

EP 129 893 A2 discloses the increase of solubility by preparing a ground mixture of a poorly soluble crystalline drug with an adsorbent. As adsorbents, solid supports, such as activated charcoal, activated clay, silica, synthetic adsorbent resin, activated alumina and other physiologically acceptable adsorbents are mentioned.

WO 2008/104852 A2 describes a pharmaceutical composition comprising an adsorbate of fenofibrate or fenofibrate having a particle size greater than or equal to about 150 µm adsorbed on an adsorbent and a process for the preparation thereof.

US 5,985,326 refers to solid dispersions in the form of co-precipitates of poorly water soluble drugs, such as tadalafil and their compositions with a pharmaceutically acceptable carrier or excipients thereof, and to the preparation of such co-precipitates.

US 6,821,975 B1 describes a free particulate form of tadalafil with a particle size of less than 40 µm. However the free form of tadalafil in a crystalline form shows a poor dissolution rate (as discussed below).

US 2007/0098804 describes a solid particulate tadalafil having a bimodal particle size distribution.

WO 2008/005039 A1 discloses a solid composite including tadalafil with the tadalafil being in intimate association with a carrier. The carriers disclosed are hydrophilic polymers such as povidone, hydroxypropyl methylcellulose, polyethylene glycol, hydroxypropyl methylcellulose phthalate, polymethacrylate, and hydroxypropyl cellulose. The carrier and the tadalafil are combined with an organic solvent to form a solution. In order to generate an intimate association between the carrier and the tadalafil, the solvent is removed by evaporation, for instance by fluidized bed drying and spray-drying.

US 2006/0286166 A1 describes particulate tadalafil having a particle size of about 200 to about 600 µm and a process for the preparation thereof. Additionally the combination of tadalafil with a solvent that can be a C₃-C₈ ketone, an aliphatic nitrile, a lower aliphatic alcohol, water, and mixtures thereof, is disclosed.

However despite the above described methods and preparations of formulations containing a poorly soluble or in water practically insoluble drug, respectively, there is a need for an improved dosage form containing a poorly soluble drug, in particular with regard to an improved dissolution profile that allows for an improved bioavailability and a good efficacy, and a process for the preparation of the dosage form.

### Summary of the invention

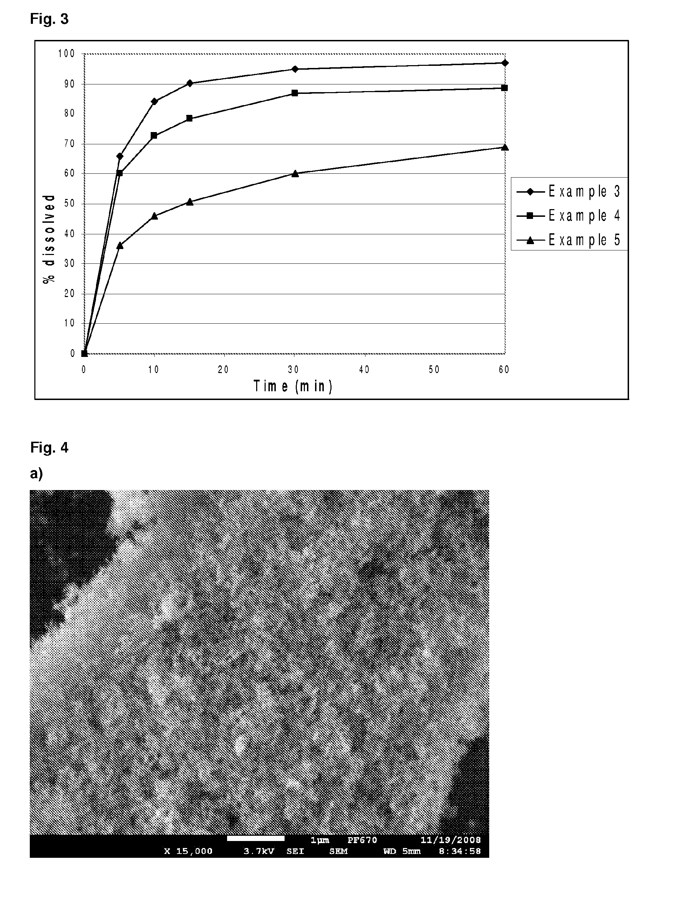

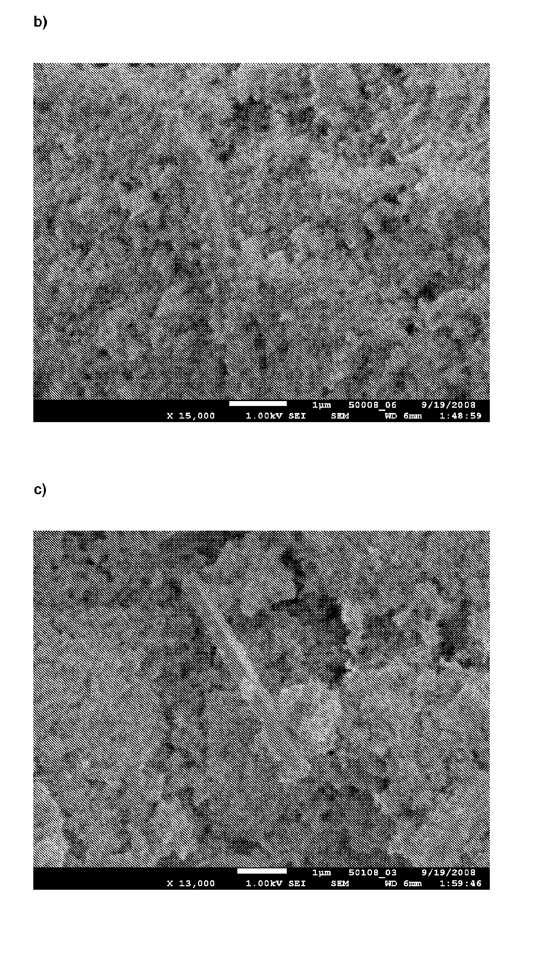

The present invention provides the following aspects, subject-matters and preferred embodiments, which respectively taken alone or in combination, further contribute to solving the object of the present invention:
(1) An adsorbate comprising an active pharmaceutical ingredient (API) being practically insoluble in water associated with a particulate and/or porous carrier.
   The adsorbate according to the present invention is thermodynamically stable and therefore forms spontaneously. Furthermore, the adsorbate is not in a kinetically trapped state, and, thus, exhibits superior chemical and/or physical stability compared to other adsorbates, e,g, to adsorbates obtained by different methods. The adsorbate according to the present invention is not just simply deposited on the surface of the respective carrier, but is stabilized by physical surface forces.
   The carrier according to the present invention is a particulate and/or porous carrier, which means that this carrier has an outer and/or inner surface onto which the API can be adsorbed. Furthermore, the carrier according to the present invention does not essentially change its morphology during the adsorbtion of the API. Preferably, the carrier is an inorganic carrier, preferably silicon dioxide and more preferably colloidal silicon dioxide. Preferred adsorbates and preferred particulate and/or porous carriers are described below. The porosity can be determined according to DIN EN 623-2, wherein the porosity is preferably at least 50 % or 60 % or 70 %, further preferred at least 80 %, further preferred at least 90 %, even further preferred at least 95 % and most preferred at least 98 %.
   With respect to the present invention, all APIs that are practically insoluble in water can be used. For instance, the API can be selected from the group consisting of proteins, peptides, nucleotides, anti-obesity drugs, nutraceuticals, corticosteroids, elastase inhibitors, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, antiretroviral drugs from the protease inhibitor class, immunosuppressants, antithyroid agents, antiviral agents, anxiolytics, sedatives, astringents, beta-adrenoreceptor blocking agents, blood products and substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, non-steroidal anti-inflammatory drugs (NSAIDs), parasympathomimetics, parathyroid calcitonin and bisphosphonates, prostaglandins, radio-pharmaceuticals, sex hormones, anti-allergic agents, stimulants and anorectics, sympathomimetics, thyroid agents, vasodilators, and xanthines. Preferably, the API within the meaning of the present invention is tadalafil, fenofibrate, cholecalciferol (vitamin D), simvastatin, NSAIDs such as naproxen and ibuprofen, megestrol acetate, ritonavir (HIV protease inhibitor), or ciclosporin, and more preferably, the API is tadalafil. The meaning of the term "practically insoluble in water" is explained below.
(2) The adsorbate according to item (1) wherein the adsorbate comprises tadalafil as API associated with a particulate and/or porous carrier.
(3) The adsorbate according to item (1) or (2) wherein tadalafil is in amorphous form. Preferably tadalafil is essentially not in the form of crystals or particles, and also preferably tadalafil is not deposited on the particulate carrier as a precipitate.
   For effectively controlling the influence affecting dissolution properties, it is feasible to provide, for instance, less than 10 %, preferably less than 7 %, more preferably less than 5 %, even more preferably less than 2 % and most preferably less than 1 % of the API being in the form of crystals or of a precipitate or particles (respectively in % by weight relative to the whole adsorbate); particularly preferably no proportion of the used API is in the form of a precipitate or particles or in the form of crystals. The true meanings of the terms "adsorbate" and, in this context, "associated with" as used herein for the present invention can be distinguished from carriers with precipitates, particles or crystals of API on their surface e.g by means of scanning electron microscopy (SEM). The morphology of the adsorbates according to the invention essentially corresponds to the morphology of the carrier material itself, which means that the API does not essentially change the morphology of the carrier material. Contrary thereto, the SEM-image of a carrier material with crystals or precipitates of API on the surface of the carrier material shows the API as objects with a different morphology on the surface of the carrier material. These crystals or precipitates or particles of API are visible separately from the carrier. Therefore, in order to find out whether an adsorbate defining a proper "association" between API and carrier according to the present invention has been obtained, a person skilled in the art can compare SEM-images of the obtained products with SEM-images from the pure carrier material (the carrier itself, i.e. without API). In preferred embodiments, in case the SEM-images of the obtained product, i.e. the adsorbate, show objects with a morphology being essentially different from the morphology of the carrier itself, the obtained product is not an adsorbate according to the present invention. Figure (fig.) 4 a shows an SEM-image of an adsorbate according to the present invention, i.e. without crystals and/or precipitates, and figures 4 b and 4 c show an SEM-image of a carrier with API in the form of a crystal.
(4) The adsorbate according to any of the preceding items wherein the carrier is silicon dioxide, preferably colloidal silicon dioxide.
(5) The adsorbate according to any of the preceding items wherein the amount of tadalafil in the adsorbate is in the range of 0.01 to 40 wt.-%, preferably in the range of 0.1 to 30 wt.-%, more preferably in the range of 1 to 30 wt.-%, and even more preferably in the range of 10 to 30 wt.-% (respectively in % by weight relative to the whole adsorbate).
(6) The adsorbate according to any of the preceding items wherein the adsorbate further contains a substance selected from the group consisting of water soluble agents, preferably the water soluble agents are selected from the group consisting of propylene glycol, triethyl citrate, polyethylene glycol 400, and polysorbate.
(7) A dosage form comprising the adsorbate according to any of items (1) - (6).
(8) The dosage form according to item (7) wherein the pharmaceutical composition contains at least one excipient, preferably the excipient is selected from the group consisting of diluents, binders, fillers, disintegrants, lubricants, sweeteners, glidants, flavourings and colouring agents;
   the fillers are selected from the group consisting of different grades of starches, such as maize starch, potato starch, rice starch, wheat starch, pregelatinized starch, fully pregelatinized starch; cellulose, such as microcrystalline cellulose or silicified microcrystalline cellulose; mannitol, erythritol; lactose, such as lactose monohydrate, lactose anhydrous, spray dried lactose or milled lactose; calcium salts, such as calcium hydrogenphosphate; sorbitol, and xylitol; particularly preferably the fillers are selected from the group consisting of pregelatinized starch, microcrystalline cellulose, silicified microcrystalline cellulose, lactose monohydrate, spray dried lactose, and milled lactose;
   the disintegrants are selected from the group consisting of carmellose calcium, carboxymethylstarch sodium, croscarmellose sodium (cellulose carboxymethylether sodium salt, crosslinked), starch, and its derivatives such as sodium starch glycolate, crosslinked polyvinylpyrrolidone (crospovidone), and low-substituted hydroxypropylcellulose; particularly preferably the disintegrants are selected from the group consisting of sodium starch glycolate and croscarmellose sodium;
   the lubricants are selected from the group consisting of stearic acid, talc, glyceryl behenate, sodium stearyl fumarate and magnesium stearate; particularly preferably the lubricant is magnesium stearate;
   the binders are selected from the group consisting of polyvinyl pyrrolidone (Povidone), copolymers of vinylpyrrolidone with other vinylderivatives (Copovidone), hydroxypropyl methylcellulose, methylcellulose, hydroxypropylcellulose, powdered acacia, gelatin, guar gum, carbomer such as carbopol, polymethacrylates and pregelatinized starch; particularly preferably the binders are selected from the group consisting of hydroxypropyl methylcellulose, hydroxypropylcellulose and copovidone;
   the diluents are selected from carbohydrates such as monosaccharides like glucose, oligosaccharides like sucrose, anhydrous lactose, lactose monohydrate or milled lactose, and sugar alcohols like sorbitol, mannitol, erythrol, and xylitol; particularly preferably the diluent is selected from the group consisting of sorbitol and milled lactose;
   the glidants are selected from the group consisting of colloidal silica, hydrophobic colloidal silica and magnesium trisilicate, such as talc; particularly preferably the glidants are selected from the group consisting of colloidal silica and hydrophobic colloidal silica; and/or
   the sweeteners are selected from the group consisting of aspartame, saccharin sodium, dipotassium glycyrrhizinate, aspartame, stevia, thaumatin, and the like; preferably the excipients are microcrystalline cellulose, silicified microcrystalline cellulose, milled lactose, croscarmellose sodium, sodium starch glycolate, hydroxypropylcellulose, colloidal silica and magnesium stearate.
(9) The dosage form according to item (7) or (8) wherein the dosage form contains microcrystalline cellulose, silicified microcrystalline cellulose, croscarmellose sodium, sodium starch glycolate, magnesium stearate, and milled lactose.
(10) The dosage form according to any of the preceding items wherein the amount of the adsorbate in the pharmaceutical composition is in the range of 1 to 95 wt.-%, preferably in the range of 5 to 90 wt.-%, more preferably in the range of 10 to 70 wt.-% and even more preferably in the range of 20 to 50 wt.-% (respectively in % by weight relative to the whole adsorbate).
(11) A process for the preparation of an adsorbate, comprising
   a) providing an active pharmaceutical ingredient (API) being practically insoluble in water, wherein the term "practically insoluble in water" defines that the solubility of the API is less or equal to 0.1 mg/ml;
   b) providing a solid support carrier in the form of particles;
   c) placing said API and said carrier into a predetermined solvent which, upon a subsequent removal thereof, allows adsorption of the API on the carrier particles but gives substantially no API-precipitate (for effectively controlling the influence affecting the dissolution properties, it is feasible to provide, for instance, less than 10 %, preferably less than 7 %, more preferably less than 5 %, even more preferably less than 2 % and most preferably less than 1 % of the total API being in the form of crystals or of a precipitate; particularly preferably no proportion of the used API is in the form of crystals or of a precipitate), and
   d) removing the solvent to form an API/carrier adsorbate. The solvent can be removed using any known methods. Preferably the solvent is removed by filtration or evaporation, preferably the solvent is removed by evaporation. Also preferably the evaporation is carried out in such a manner that the organic solvent is evaporated slowly, suitably during a period of at least 30 minutes, more preferably during a period of at least 2 hours. Slowly removing the solvent has the benefit that uneconomic, complex and laborious process steps, which would otherwise be necessary to achieve a fast removal, are not necessary. Additionally, the slow removal of the solvent leads to the formation of adsorbates having improved properties with respect to stability and solubility of the API.
(12) The process according to item (11) wherein the API is tadalafil and/or wherein the carrier is an inorganic carrier, such as silicon dioxide, preferably colloidal silicon dioxide.
(13) The process according to item (11) or (12) wherein the solvent is a non-polar solvent, preferably the solvent is a chlorinated hydrocarbon, preferably dichloromethane. The solvent can also be a mixture of different solvents.
(14) The process according to any of items (11) to (13) wherein at least one water soluble agent is added at any time during the process, preferably prior to step (d).
(15) The process according to any of items (11) to (14) wherein steps (a) to (c) are accomplished by dissolving the API in the solvent, preferably providing a clear solution, and then combining the resulting solution with a solid support carrier.
(16) A process for the preparation of a dosage form comprises formulating an adsorbate of an active pharmaceutical ingredient (API) being practically insoluble in water, associated with a particulate or porous carrier, preferably a porous particulate carrier. For the definition of the terms "adsorbate" and, in this context, "associated with", reference is made to the above, especially item (3). With regard to the term "porosity", reference is made to the above, especially item (1). As noted above, the term "practically insoluble in water" typically defines that the solubility of the API is less or equal to 0.1 mg/ml.
   In general, an adsorbate may be formulated and incorporated into solid dosage forms by a wide spectrum of techniques commonly used in the art. The process according to the present invention, preferably used for adsorbates with high drug loadings (as, e.g., for tadalafil adsorbates), comprises the steps of:
   a) providing a mixture of the adsorbate, formed by the API being practically insoluble in water on or inside the particulate or porous carrier, and at least one excipient,
   b) fine-milling of the mixture of step (a) and preferably mixing the fine milled mixture of step (a) with excipients, preferably with excipients as defined in item (8), or co-milling or fine dispersing of the mixture of step (a) with excipients, preferably with excipients as defined in item (8), preferably in an impact-mill,
   c) formulation of the mixture of step (b) into a dosage form, preferably by a dry formulation technique.
(17) In general, an adsorbate may be simply blended with excipients, for instance with excipients as decribed in item (8), and subjected to processes for the preparation of a dosage form commonly used in the art. In a preferred embodiment of the process as defined in item (16), preferably when preparing dosage forms comprising adsorbates with high drug loadings, the excipient in step (a) is a hydrophilic excipient, and more preferably the excipient is selected from the group consisting of lactose, microcrystalline cellulose, silicified microcrystalline cellulose, croscarmellose sodium and sodium starch glycolate.
(18) In general, adsorbates may be processed like API in pure form and can be formulated using wet or dry granulation processes, direct compression, or any pelletization techniques. In a preferred embodiment of the process according to item (16) or (17), preferably used for dosage forms comprising adsorbates with high drug loadings, the preferred dry formulation technique in step (c) is dry granulation or direct compression, preferably in case of low dosage preparations, direct compression is employed, and in case of high dosage preparations, dry granulation is employed.
(19) The process according to any one of items (16) to (18) wherein any further excipient as defined in item (8) are added, preferably after step (b).
(20) The process according to item (19) wherein a further excipient is selected from sodium starch glycolate, croscarmellose sodium and magnesium stearate.
(21) The process according to any one of items (16) to (20) wherein the prepared adsorbate is essentially free of crystals or precipitate of the API, defined by less than 10 %, preferably less than 7 %, more preferably lass than 5 %, even more preferably less than 2 % and most preferably less than 1 % of the API being in the form of crystals or of a precipitate (respectively in % by weight relative to the whole adsorbate); particularly preferably no proportion of the used API is in the form of crystals or of a precipitate.
(22) The process according to any one of items (16) to (21) wherein under (a), the adsorbate is an adsorbate according to any of items (1) to (6) for the preparation of a dosage form according to any one of items (7) to (10).
(23) The process according to any one of items (16) to (21) wherein under (a) the adsorbate is an adsorbate prepared according to any one of items (11) to (16).
(24) Use of an adsorbate according to any one of items (1) to (6) for the preparation of a dosage form.
(25) The dosage form according to any one of items (7) to (10) containing tadalafil, for use in the treatment of pulmonary arterial hypertension, hypercholesterolemia, or sexual dysfunctions, such as male erectile dysfunction, or a dosage form according to any of items (7) to (10) containing non-steroidal anti-inflammatory drugs, immunosuppresants, and/or antiretroviral drugs from the protease-inhibitor class for use in the treatment of HIV infections and/or AIDS.

### Detailed description of the invention

The present invention is now described in more detail by preferred embodiments and examples, which are however presented for illustrative purpose only and shall not be understood aslimiting the scope of the present invention in any way.

It was surprisingly found that the method according to the present invention allows providing true adsorbates comprising an API, in particular an API being practically insoluble in water, e.g. tadalafil, fenofibrate, cholecalciferol (vitamin D), simvastatin, NSAIDs such as naproxen and ibuprofen, megestrol acetate, ritonavir (HIV protease inhibitor), or ciclosporin, associated with a carrier, provided that a particulate and/or porous carrier is used. However, given the general applicability of the presently disclosed concept when practically insoluble API are used, the API can also be selected from the group consisting of proteins, peptides, nucleotides, anti-obesity drugs, nutraceuticals, corticosteroids, elastase inhibitors, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, antiretroviral drugs from the protease inhibitor class, immunosuppressants, antithyroid agents, antiviral agents, anxiolytics, sedatives, astringents, beta-adrenoreceptor blocking agents, blood products and substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, non-steroidal anti-inflammatory drugs (NSAIDs), parasympathomimetics, parathyroid calcitonin and bisphosphonates, prostaglandins, radio-pharmaceuticals, sex hormones, anti-allergic agents, stimulants and anorectics, sympathomimetics, thyroid agents, vasodilators, and xanthines. Preferably, the API within the meaning of the present invention is selected from the group consisting of tadalafil, fenofibrate, cholecalciferol (vitamin D), simvastatin, NSAIDs such as naproxen and ibuprofen, megestrol acetate, ritonavir (HIV protease inhibitor), or ciclosporin, and more preferably, the API is tadalafil. As a significant contribution of increasing solubility of APIs being, as such, practically insoluble in water, the active ingredient associated with the carrier is essentially not in the form of crystals or in the form of precipitates or particles. For effectively controlling the influence affecting dissolution properties, it is favorable to provide, for instance, less than 10 %, preferably less than 7 %, more preferably less than 5 %, even more preferably less than 2 % and most preferably less than 1 % of the total API being in the form of crystals or of a precipitate; particularly preferably no proportion of the used API is in the form of crystals or of a precipitate. When carrying out the method according to the present invention, the API is well adsorbed on the outer, optionally also the inner surface of a carrier from a solution of the API in a solvent. In particular, observing proper compatibility between the relevant components used (API, particulate carrier and solvent) in terms of avoiding precipitation or crystal formation of the API during the solvent removal, the adsorption process can be made exothermal and driven by the affinity of the API towards the carrier surface. Using the invention, the majority and, when compatibility between the relevant components is observed, essentially all and possibly even the total amount of the API in the solution will be adsorbed on, and homogenously cover the particulate carrier and does not form precipitates or crystals which might eventually deposit on the carrier upon the solvent removal. As a consequence, the API shows an improved dissolution rate that allows for an improved bioavailability and an improved efficacy.

The dissolution rate of an API from an adsorbate is higher, compared to the dissolution rate of an API from precipitates, which, in turn, is higher, compared to the dissolution rate of an API in a crystalline form. It has been unexpectedly found that the adsorbates according to the present invention have a higher dissolution rate than API precipitates, even if the latter is deposited on a particulate carrier. The adsorbates according to the present invention can therefore be used to prepare pharmaceutical dosage forms having improved properties, such as exhibiting an improved dissolution rate, an improved bioavailability and an improved efficacy. In the treatment of disorders, such as pulmonary arterial hypertension or sexual dysfunctions, such as male erectile dysfunctions, hypercholesterolemia, HIV infections or AIDS, a rapid achievement of the maximum blood concentration, along with a greater prospect for a rapid onset of therapeutic effect, is desirable. The rapid achievement of the maximum blood concentration can be reached by an improved dissolution rate, which, in turn, leads to an improved bioavailability and therefore an improved efficacy.

It has furthermore been found that it is advantageous to use a non-polar solvent in a combination with a an API, being practically insoluble in water, and that it is more advantageous to use a chlorinated solvent, and that it is in particular advantageous to use dichloromethane as the solvent, particularly if tadalafil is used as the API. Other non polar organic solvents are also suitable and can be generally referred to as solvents non-miscible with water at room temperature, including but not limited to liquid hydrocarbons, halogenated hydrocarbons, alcohols, esters, ethers, and ketones, respectively; each of them being non-miscible with water. Without being bound to any theory, it appears that compatibility between such types of solvent and APIs being practically insoluble in water can be suitably balanced with the solid particulate and/or porous carrier.

In addition, the formation of the adsorbate according to the present invention does not depend on a rapid removal of the solvent. Therefore the process according to the present invention beneficially avoids uneconomic, complex and laborious steps and equipments (for example spraying or fluid bed techniques) which would otherwise be necessary for a fast evaporation of the solvent. In addition, slow evaporation leads to an equilibrated adsorption process leading to the formation of adsorbates having improved properties. For instance, in case of fast solvent evaporation the substance is often in an unequilibrated physical state with deteriorated physical and chemical stability. Slow evaporation results in an equilibrated physical state with superior physical and chemical stability.

It has also been found that the method according to invention allows preparing adsorbates having a high amount of the API on the carriers.

In addition, it has been found that co-milling, preferably impact-milling, of the adsorbates according to the present invention with one or more excipients when preparing dosage forms according to the invention increases the total amount of the API released from the dosage form and therefore increases the bioavailability of the dosage form compared to prior art formulations.

In one particular aspect, the present invention relates to an adsorbate comprising tadalafil associated with a particulate and/or porous carrier.

The expression "adsorbate", as used herein, specifies that the API is, preferably evenly, distributed on the inner and/or outer surface of the particulate carrier and preferably substantially all of the API is associated with a surface of the carrier. "Substantially all of the API" within the meaning of the present invention denotes more than 75 %, preferably more than 80 %, more preferably more than 90 %, even more preferably more than 95 % and most preferably more than 98 %, all of the total API. "Association" in this context means that the API and the surface of the carrier form an adsorbate. During preparation of the adsorbate, the adsorption of API on the molecular level is influenced by the surface of the carrier, which means that the API is adsorbed by the carrier and that the structure of the adsorbed API on the surface is determined by the surface of the carrier and is, therefore, different from the structure of self-associated API molecules without the proximity of the carrier surface. The structure of the API in the adsorbate is determined by the combination of surface-API molecule forces as opposed to the structure of the co-precipitate, crystals or otherwise coarse particulate API, where the structure is determined solely by intermolecular forces between API molecules. Without being bound to any theory, it may be assumed that the API molecules which are present in the solvent may not readily associate with API molecules which are already present on the surface of the carrier and do not form crystals, precipitates or otherwise coarse particulate API. The resulting physical status of the API may favor a better dissolution profile of the adsorbate comprising the API compared to the dissolution profile of an adsorbate comprising the API in a particulate form, wherein the API is microscopically not properly associated with the carrier substance. Preferably the API does not form coarse particles, larger crystals or precipitates which would form a mere mixture with the carrier. The true adsorbate within the meaning of the present invention can be distinguished from carriers with precipitates or crystals of API on their surface e.g. by means of scanning electron microscopy (SEM). The morphology of the adsorbates according to the invention essentially corresponds to the morphology of the carrier material itself, which means that the API does not essentially change the morphology of the carrier material. Contrary thereto, the SEM-image of a carrier material with crystals, precipitates, or coarser particles of API on the surface of the carrier material shows the API as objects with a different morphology on the surface of the carrier material. Therefore, in order to find out whether an adsorbate according to the present invention has been obtained, a person skilled in the art can compare SEM-images of the obtained products with SEM-images from the pure carrier material (the carrier itself, i.e. without API). In preferred embodiments, in case the SEM-images of the obtained product, i.e. the adsorbate, show objects with a morphology being essentially different from the morphology of the carrier itself, the obtained product is not an adsorbate according to the present invention.

In the adsorbate according to the invention, the API being practically insoluble in water, such as tadalafil, is preferably mainly in amorphous form, preferably essentially all of the API, e.g. all of the tadalafil, is in amorphous form. This is the case if an analysis of the adsorbate or the dosage form using suitable methods known to a person skilled in the art, such as differential scanning calorimetry (DSC) or X-ray powder diffraction (XRPD), does not show reflexes or melting transition of the crystalline form of the respective API. The characteristic reflexes of the respective API can be derived by a person skilled in the art from data bases or simply by measuring the API starting material in crystalline form. Peak lists of the characteristic reflexes of tadalafil are for instance described in US2006/0111571. The expression "adsorbate" furthermore relates to an isolated carrier (dry state) containing the API adsorbed on its inner or/and outer surface. Such an adsorbate can be used for the preparation of dosage forms as described herein.

It is also preferred that the carrier has a high BET-surface area, preferably the BET-surface area is at least 1 m²/g, even more preferably the BET-surface area is in a range of from 10 to 1000 m²/g, even more preferably of from 20 to 500 m²/g and most preferably of from 100 to 450 m²/g. The determination of the BET-surface area of the carrier can be carried out according to the method as described in the article: J. Am. Chem. Soc. 60, 309 (1938). Additionally preferred, the carriers with the defined BET-surfaces have a porosity as defined below.

The carrier to be used according to the present invention can be any particulate and/or porous carrier as defined above. Preferably, the carrier is an inorganic carrier. Further preferred, the carrier does not essentially change its morphology before, during and after the adsorption of the API. Preferably the carrier is silicon dioxide, more preferably colloidal silicon dioxide. For example, Aerosil^{®} 90, 130, 150, 200 or 380 or Aerosil^{®} OX 50, EG 50 or TT 600 (Evonik Degussa GmbH, Germany) can be used. Preferably Aerosil^{®} 200 or 380 can be used. Also preferably, the carrier is silicified microcrystalline cellulose, suitable carrier material can e.g. be obtained from JRS Pharma, sold under the trade name PROSOLV^{®} SMCC.

Additionally preferred, the carrier has a porosity of at least 50 %, preferably of at least 60 %, more preferably of at least 70 %, further preferably of at least 80 %, further preferably of at least 90 %, even further preferably of at least 95 % and most preferably of at least 98 % as determined by DIN EN 623-2. The term "porosity" as used herein refers to the open pore porosity, which can be determined using the aforementioned method. The open pores of the carrier will typically be accessible to the solvent containing the API during the process for preparation of the adsorbates.

The adsorbates according to the present invention can contain a high amount of API, in particular tadalafil, adsorbed on the surface of the carrier. Preferably the amount of API in the adsorbate is in the range of from 0.01 to 40 wt.-%, preferably in the range of from 0.1 to 30 wt.-%, more preferably in the range of from 1 to 30 wt.-%, and even most preferably in the range of from 10 to 30 wt.-%.

Additionally, the adsorbates according to the present invention preferably contain water soluble agents. These water soluble agents are soluble in the solvent used for the preparation of the adsorbates according to the present invention. Preferred water soluble agents are propylene glycol, triethyl citrate, polysorbates and polyethylene glycol 400.

The present invention also relates to a dosage form comprising the adsorbate according to the invention.

In addition to the adsorbate, the dosage form according to the invention preferably contains at least one excipient, more preferably at least two excipients, even more preferably at least three excipients, and even more preferably at least four excipients. Suitable excipients are known in the art, preferably the excipients are selected from the group consisting of diluents, binders, fillers, disintegrants, lubricants, sweeteners, glidants, flavourings and colouring agents, preferably the fillers are selected from the group consisting of different grades of starches, such as maize starch, potato starch, rice starch, wheat starch, pregelatinized starch, fully pregelatinized starch; cellulose, such as microcrystalline cellulose or silicified microcrystalline cellulose, mannitol, erythritol; lactose, such as lactose monohydrate, lactose anhydrous, spray dried lactose or milled lactose; calcium salts, such as calcium phosphate, calcium hydrogenphosphate, calcium carbonate, sorbitol, and xylitol, particularly preferably the fillers are selected from the group consisting of pregelatinized starch, microcrystalline cellulose, silicified microcrystalline cellulose, lactose monohydrate, spray dried lactose, and milled lactose;
the disintegrants are selected from the group consisting of carmellose calcium, carboxymethylstarch sodium, croscarmellose sodium (cellulose carboxymethylether sodium salt, crosslinked), starch, such as sodium starch glycolate or com starch, crosslinked polyvinylpyrrolidone (crospovidone), and low-substituted hydroxypropylcellulose, particularly preferably the disintegrants are selected from the group consisting of sodium starch glycolate and croscarmellose sodium;
the lubricants are selected from the group consisting of stearic acid, glyceryl behenate, talc, sodium stearyl fumarate and magnesium stearate, particularly preferably the lubricant is magnesium stearate;
the binders are selected from the group consisting of polyvinyl pyrrolidone (Povidone), copolymers of vinylpyrrolidone with other vinylderivatives (Copovidone), hydroxypropyl methylcellulose, methylcellulose, hydroxypropylcellulose, powdered acacia, gelatin, guar gum, carbomer such as carbopol, polymethacrylates and starch, particularly preferably the binders are selected from the group consisting of hydroxypropyl methylcellulose, hydroxypropylcellulose and copovidone;
the diluents are selected from carbohydrates such as monosaccharides like glucose, oligosaccharides like sucrose, anhydrous lactose, lactose monohydrate or milled lactose, and sugar alcohols like sorbitol, mannitol, erythrol, and xylitol, particularly preferably the diluent is selected from the group consisting of sorbitol and milled lactose;
the glidants are selected from the group consisting of colloidal silica, hydrophobic colloidal silica and magnesium trisilicate, such as talcum, particularly preferably the glidants are selected from the group consisting of colloidal silica and hydrophobic colloidal silica; and/or the sweeteners are selected from the group consisting of aspartame, saccharin sodium, dipotassium glycyrrhizinate, aspartame, stevia, thaumatin, and the like, further preferably the excipients are microcrystalline cellulose, croscarmellose sodium, hydroxypropylcellulose and colloidal silica.

In a preferred embodiment of the invention, the dosage form contains at least one, more preferably at least two, even more preferably at least three and even more preferably at least four of the excipients from the group consisting of microcrystalline cellulose, croscarmellose sodium, sodium starch glycolate, magnesium stearate, and, lactose. In an even further preferably embodiment, the dosage form contains all of the aforementioned excipients.

It is additionally preferred that the dosage form according to the invention contains an amount of adsorbate in the range of 1 to 95 wt.-%, more preferably in the range of 5 to 90 wt.-%, even more preferably in the range of 10 to 70 wt.-% and most preferably in the range of 20 to 50 wt.-%.

The present invention also relates to a process for the preparation of the adsorbates according to the invention. The process for the preparation of an adsorbate comprises the steps of:
a) providing an API being practically insoluble in water, wherein the term "practically insoluble in water" defines that the solubility of the API is less or equal to 0.1 mg/ml;
b) providing a solid support carrier in the form of particles;
c) placing said API and said carrier into a predetermined solvent which, upon a subsequent removal thereof, allows adsorption of the API on the outer and/or inner surface of the carrier particles but gives substantially no API-precipitate, and
d) removing the solvent to form an API-carrier adsorbate. The solvent can be removed using any known methods. Preferably, the solvent is removed by filtration or evaporation, more preferably the solvent is removed by evaporation, most preferably by slow evaporation. In addition, slow evaporation leads to an equilibrated adsorption process leading to the formation of adsorbates having improved properties. For instance, in case of fast solvent evaporation the substance is often in an unequilibrated physical state with deteriorated physical and chemical stability. Slow evaporation results in an equilibrated physical state with superior physical and chemical stability.

The steps (a) to c) as well as the steps being comprised in step (c) can be carried out in any order; they can be accomplished in one or more common steps. Preferably the API is dissolved in the solvent, and then, the resulting solution is combined with a solid support carrier.

The API that is used in the process according to the present invention, when determined as a property as such (i.e. not in the adsorbate form), is practically insoluble in water. The term "practically insoluble in water" within the meaning of the present invention denotes a solubility of less or equal to 0.1 mg/ml as defined in the US or European Pharmacopoeias, such as U.S. Pharmacopoeia (U.S.P.)XXI (page 1441). Preferably, the API to be used In the process is tadalafil. The carriers which are suitable for the process according to the invention are defined above.

In the process of preparing adsorbates according to the invention, selection of a suitable solvent in relation of both a given carrier and an API to be used has been found to be significant. To determine a suitable solvent/carrier combination, the following general reference test can be carried out: A desired type and amount of an API is dissolved in a solvent to be tested and combined with an appropriate carrier with sufficient surface area as determined by BET. Preferably, the amount of carrier is chosen in order that at least 500 m² of carrier surface / g API, more preferably at least 1000 m² of carrier surface / g API, even more preferably at least 1500 m² of carrier surface / g API, further preferably at least 2000 m² of carrier surface / g API, most preferably at least 3000 m² of carrier surface / g API is provided. After the addition of the carrier to the solution, the concentration of the free API in the solution is lowered in the case of suitable solvent/carrier combination. If no precipitates are formed, e.g. during the solvent removal process, the combination of solvent/carrier is suitable and will result in adsorbates according to the invention. As described above, the obtained adsorbate according to the present invention can for instance be analyzed by SEM-imaging.

In case there is no lowering of the free API concentration after addition of the carrier, the solvent/carrier combination is not suitable for the desired API, and the API will not be properly adsorbed on the carrier. Example 1 exemplifies such a test for preparing a tadalafil adsorbate.

The organic solvent can be removed by any method known to a person skilled in the art. Preferably, the solvent is removed by filtration or evaporation, more preferably the solvent is removed by evaporation. The organic solvent can be removed by evaporation at normal atmosphere or at a partial vacuum. The evaporation is preferably carried out in such a manner that the organic solvent is evaporated slowly, suitably during a period of at least 15 minutes, further preferred at least 30 minutes, more preferably during a period of at least 2 hours. This slow evaporation process has the benefit that uneconomic, complex and laborious process steps, which would otherwise be necessary for a fast evaporation, can be avoided. Additionally, the slow evaporation process leads to a better formation of the adsorbate. Moreover, slow evaporation leads to an equilibrated adsorption process leading to the formation of adsorbates having improved properties. For instance, in case of fast solvent evaporation the substance is often in an unequilibrated physical state with deteriorated physical and chemical stability. Slow evaporation results In an equilibrated physical state with superior physical and chemical stability.
Preferably the solvent to be used in the process according to the invention is a non-polar organic solvent, preferably the solvent is a chlorinated hydrocarbon, and preferably dichloromethane is used. Preferably the aforementioned solvents are used in a combination with silicon dioxide or microcrystalline cellulose, preferably silicon dioxide, as the carrier. The solvent can also be also a mixture of solvents.

In another preferred embodiment of the invention, at least one water soluble agent as defined above is added at any time during the process, preferably prior to step (d).

The present invention furthermore relates to a process for the preparation of a dosage form according to the invention comprising the steps of:
a) providing a mixture of the adsorbate according to the invention and the excipient/-s,
b) fine-milling of the mixture of step (a) and preferably mixing the fine milled mixture of step (a) with excipients, preferably with excipients as defined in item (7), or co-milling or fine dispersing of the mixture of step (a) with excipients, preferably with excipients as defined in item (7), preferably In an impact-mill,
c) formulation of the mixture of step (b) into a dosage form. For step (c), preferably a dry granulation process is used. In case of formulations with a concentration of adsorbate of 20 % (wt./wt.) or less (amount of adsorbate per amount of dosage form), preferably direct compression is employed. If concentration of adsorbate is more than 20 % preferably a dry granulation process is employed.

According to the invention, one or more excipients can be used as described above. Preferred excipients are also described above. In a preferred embodiment, at least one excipient is used, preferably the excipient is a fine grade hydrophilic excipient, and more preferably said excipient is selected from the group consisting of lactose, microcrystalline cellulose, and croscarmellose sodium. Most preferably at least microcrystalline cellulose and croscarmellose sodium are used.

The mixture resulting from step (a) is preferably subjected to a milling step. Preferably the mixture is fine-milled, and more preferably impact-milled. From the resulting particles, 90 % are equal to or smaller than 500 µm, preferably 90 % of the resulting particles are in the range of from 50 to 500 µm, more preferably in the range of from 100 to 400 µm, even more preferably in the range of from 200 to 300 µm, and most preferably in the range of from 220 to 260 µm.

It is additionally preferred that after step (b), at least one further excipient as defined herein is added, preferably sodium starch glycolate, croscarmellose sodium and magnesium stearate are added. After step (b) and prior to step (c), additional mixing and/or milling steps can be carried out.

As a last step, the mixture is formulated into a dosage form. The formulations of the present invention may be prepared by well known technological processes. Preferably any dry formulation technique that is known to a person skilled in the art can be used, such as dry granulation or direct compression, preferably in case of formulations with a concentration of adsorbate of 20 % or less (wt.lwt.), direct compression is employed, and in case of formulations with higher adsorbate concentrations (20 % (wt./wt.) or more), dry granulation is preferred. Direct compression, as well as dry granulation, relative to other process technologies, have the advantage that they eliminate the need for water or alcohol and can further eliminate the need for still other solvents and even for any liquid substances, and therefore allows the formulation of solvent sensitive drugs. Additionally, the omission of water or alcohol or other solvents further avoids crystallization of the API, and therefore additionally contributes to an improved dissolution rate compared to the dissolution rate of the API being present in a crystalline form. That is, this aspect of the present invention is valuable not only for an API having a low solubility in water (indicative for a hydrophobic nature of the selected API), but also for an API being sensitive to solvents in general and even more for an API with a tendency of forming precipitates or crystallizing in solvents (particularly water or alcohol, such as methanol or ethanol). Furthermore direct compression is the fastest and most cost effective way of tablet preparation. In order to carry out direct compression, a person skilled in the art can choose a suitable apparatus. In order to tablet a dry granulate, a person skilled in the art can choose a suitable apparatus, for instance, a rotary tabletting machine can be used. A dosage form according to the present invention can be any pharmaceutically suitable dosage form, preferably in a solid form, including tablets, capsules (soft or hard capsules), caplets, lozenges, and sachets. A dosage form according to the present invention is preferably in the form of a tablet. Furthermore the dosage form can comprise a coating. Suitable coatings are known in the art, e.g. film coatings, usually applied for colouring purposes.

The present invention furthermore refers to the use of an adsorbate according to the invention for the preparation of a dosage form.

The present invention also relates to the dosage form according to the invention for use in the treatment of pulmonary arterial hypertension, or sexual dysfunctions, such as male erectile dysfunction.

### Description of the figures

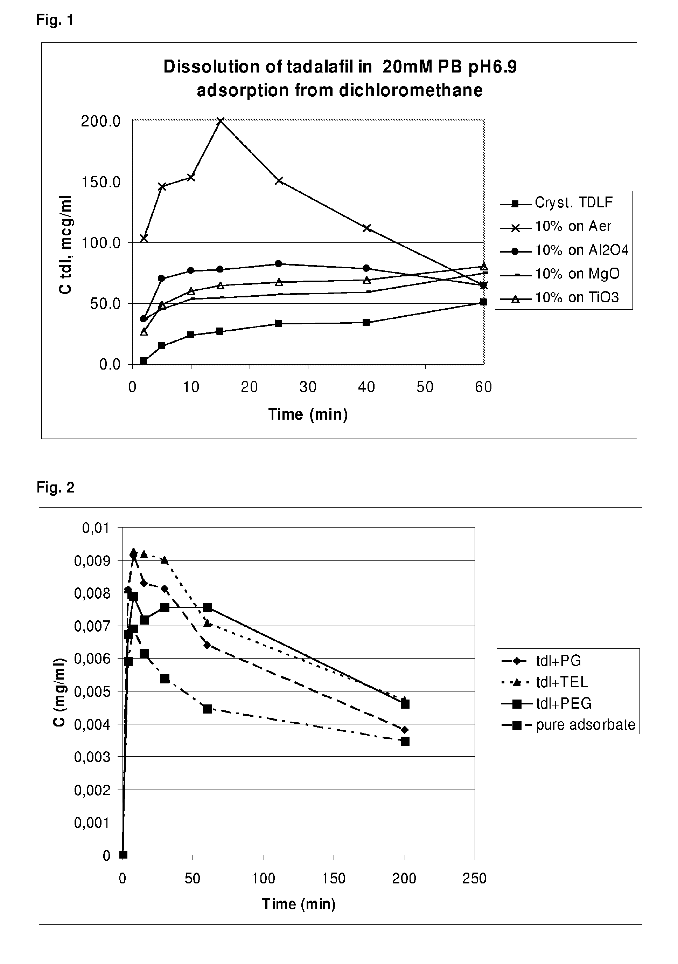

Figure 1 shows the dissolution of crystalline tadalafil and precipitates of tadalafil in comparison with an adsorbate of tadalafil from dichloromethane according to the invention.
   The carriers comprising tadalafil have been prepared as described in example 1. Figure 1 shows the dissolution profile of the following samples: 10 % tadalafil on Aerosil^{®} 380 from dichloromethane, 10 % tadalafil on MgO from dichloromethane, 10 % tadalafil on Al₂O₃ from dichloromethane, and 10 % tadalafil on TiO₂ from dichloromethane in 20 mM phosphate buffer pH 6.9.
Figure 2 shows the dissolution of the pure tadalafil-adsorbate ("pure adsorbate") and tadalafil adsorbates ("tdl") with different hydrophilic additives like propylene glycol (PG), triethyl citrate (TEL), and polyethylene glycol 400 (PEG). The ratio of tadalafil : Aerosil : additive is 1 : 5 : 0.3 wt/wt. The preparation of the adsorbates using water soluble agents is described in examples 2 and 12.
Figure 3 shows the dissolution from samples as prepared in examples 3, 4 and 5. An A2 dissolution apparatus (1000 mL, 50 rpm) is used as described in US Pharmacopeia (USP), the dissolution media is water with of 0.5% of sodium dodecyl sulphate (SDS). Examples 3 and 4 show a faster dissolution profile. The compositions used in these examples are prepared by impact-milling of tadalafil-adsorbate with excipients. The composition according to example 5 is prepared without impact-milling.
Fig. 4 shows an SEM-image of an adsorbate according to the present invention, i.e. without precipitates and/or crystals (fig. 4 a), and an SEM-image of a carrier with API in form of a crystal (figures 4 b and 4 c).

### Examples

### Example 1: Preparation of adsorbates (according to the invention) and precipitates (for comparison reasons)

0.1 g of tadalafil was dissolved in 20 ml of dichloromethane. 5 ml of the resulting solution was added to 0.25 g of a solid support (Aerosil^{®} 380, Al₂O₃, MgO, TiO₂). Subsequently the solvent was removed by evaporation.

The samples were analysed using differential scanning calorimetry (DSC), and X-ray powder diffraction (XRPD). Using XRPD it was confirmed that in all products, the API (adsorbate or precipitates) was amorphous. The use of dichloromethane as the solvent for tadalafil and Aerosil as the carrier gives the adsorbate according to the invention. The use of MgO, Al₂O₃ and TiO₂ as carriers results in precipitates (see figure 1).

In all of the above products, the API on the carrier showed markedly improved dissolution rate over the crystalline tadalafil. The adsorbate, 10 % of tadalafil on Aerosil^{®} 380 from dichloromethane, showed markedly improved dissolution rate over other forms, especially precipitates (cf. rest of the samples). The results are shown in table 1 and figure 1. Moreover, the tadalafil dissolution rate from the adsorbate, having an adsorbed proportion of 10 % of tadalafil on Aerosil^{®} 380 (weight-% relative to total adsorbate), from dichloromethane solvent showed markedly improved dissolution rate compared to the dissolution rate of the adsorbate, having 10 % of tadalafil on Aerosil^{®} 380, adsorbed from tetrahydrofuran solvent (not shown).

**Table 1. Tadalafil, adsorbate and precipitates from dichloromethane**

| | Amount dissolved (µg/ml) | | | | |
|---|---|---|---|---|---|
| Time (min) | Crystalline tadalafil | 10% on Aerosil 380 | 10% on Al2O3 | 10% on MgO | 10% on TiO2 |
| 2 | 2.9 | 103.3 | 37.1 | 37.0 | 27.2 |
| 5 | 14.6 | 146.0 | 70.8 | 45.3 | 49.3 |
| 10 | 24.2 | 153.5 | 76.8 | 54.0 | 60.3 |
| 15 | 27.0 | 200.3 | 78.0 | 54.4 | 65.3 |
| 25 | 32.9 | 151.1 | 82.3 | 57.7 | 68.0 |
| 40 | 34.4 | 112.3 | 78.9 | 59.4 | 69.8 |

### Example 2: Use of water soluble agents

The adsorbates according to the present invention have specific surface characteristics depending on the molecule being adsorbed (e.g. tadalafil is a lipophilic molecule - this results in a lipophilic surface). Surface characteristics may be modified by inclusion of water soluble agents, such as propylene glycol (PG), triethyl citrate (TEC), or polyethylene glycol 400 (PEG). This modification results in a better wettability of the adsorbate and additionally the acceleration of the dissolution of the API (compare figure 2).
1.6 g of tadalafil was dissolved in 300 ml of dichloromethane. Water soluble agent (0.48 g) was added, followed by addition of Aerosil 380 (10 g). The mixture was stirred at room temperature for 30 minutes and then the solvent was removed by evaporation.

### Example 3: Composition and preparation of tadalafil film-coated tablets

| Component | Composition (% wt./wt.) |
|---|---|
| Tadalafil-adsorbate | 30.6 |
| Milled lactose | 45.7 |
| Microcrystalline cellulose | 9.2 |
| Croscarmellose sodium | 5.5 |
| Sodium starch glycolate | 8.3 |
| Magnesium stearate | 0.7 |

Tadalafil-adsorbate (which contains 15 % of tadalafil adsorbed onto Aerosil^{®} 380) is mixed with milled lactose, microcrystalline cellulose and croscarmellose sodium. The mixture is milled twice on impact mill (screen: 0.25 mm, 5000 rpm, hammer). 2/3 of sodium starch glycolate is added, mixed for 2 minutes, magnesium stearate is added and mixed for 1 minute. Thus obtained mixture is slugged and milled on an oscilating bar screening mill: first through a 2.0 mm screen and finally through a 1.0 mm screen. A dry granulate is obtained: 1/3 of sodium starch glycolate is added and mixed for 2 minutes. The final blend was tabletted by using a rotary tabletting machine to yield tablets with a mass of 436 mg. The batch size was 800 g.

The tablets were further film-coated for colouring purposes. A standard hypromellose based coating was used. The film-coating was accomplished in pan coater with a perforated drum.

### Example 4: Composition and preparation of tadalafil tablets

| Component | Composition (% wt./wt.) |
|---|---|
| Tadalafil-adsorbate | 30.6 |
| Microcrystalline cellulose | 54.9 |
| Croscarmellose sodium | 5.5 |
| Sodium starch glycolate | 8.3 |
| Magnesium stearate | 0.7 |

Tadalafil-adsorbate (which contains 15 % of tadalafil adsorbed onto Aerosil^{®} 380) is mixed with microrystalline cellulose and croscarmellose sodium. The mixture is milled twice on an impact mill (screen: 0.25 mm, 5000 rpm, hammer). 2/3 of sodium starch glycolate is added, mixed for 2 minutes, magnesium stearate is added and mixed for 1 minute. The obtained mixture is slugged and milled on an oscilating bar screening mill: first through a 2.0 mm screen and finally through a 1.0 mm screen. A dry granulate is obtained: 1/3 of sodium starch glycolate is added and mixed for 2 minutes. The final blend was tabletted by using a rotary tabletting machine to tablets with a mass of 436 mg. The batch size was 800 g.

### Example 5: Composition and preparation of tadalafil tablets

| Component | Composition (% wt./wt.) |
|---|---|
| Tadalafil-adsorbate | 33.9 |
| Milled lactose | 25.9 |
| Microcrystalline cellulose | 35.9 |
| Croscarmellose sodium | 3.5 |
| Magnesium stearate | 0.8 |

Tadalafil-adsorbate is mixed with milled lactose, microcrystalline cellulose and croscarmellose sodium. Magnesium stearate is added and mixed for 1 minute. Mixture is slugged and milled on oscilating bar screening mill: first through a 2.0 mm screen and finally through a 1.0 mm screen. A dry granulate is obtained and tabletted using a rotary tabletting machine to yield tablets with a mass of 400 mg. The batch size was 800 g.

Adsorbates with hydroplilic additives and samples described in Examples 3 - 5 were further tested for dissolution.

Examples 3-5 show that the adsorbate according to the invention can be incorporated into tablets by means of processes applicable in a tablet manufacturing. The resulting dosage forms have good properties with respect to the dissolution of the API. However the dissolution profile can be further improved by fine co-milling the adsorbate, particularly with fine grade hydrophilic excipients. This results in the complete release of the API from the tablets (see examples 3 and 4). Example 5 is prepared by mere physical mixing, however without a fine co-milling. The dissolution profiles of examples 3 - 5 are provided in figure 3, the dissolution enhancement of the fine-milled samples is evident.

### Example 6: Tadalafil - Aerosil^{®} 200 adsorbate

Tadalafil (2 g) was suspended in dichloromethane (500 ml). The mixture was heated to reflux and stirred at this temperature for 30 minutes. When all of the material dissolved, the clear solution was cooled to 25 °C and Aerosil^{®} 200 (20 g) was added. The suspension was stirred at 25 °C for 1 hour. The solvent was then evaporated under a reduced pressure at 40 °C. The product was milled through a 0.50 mm sieve and then dried overnight in vacuum at 60 °C to give 20.6 g of the adsorbate.

### Example 7: Tadalafil - Aerosil^{®} 200 adsorbate

Tadalafil (50.4 g) was suspended in dichloromethane (7.5 I). The mixture was heated to reflux and stirred at this temperature for 30 minutes. When all of the material dissolved, the clear solution was cooled to 25 °C and Aerosil^{®} 200 (430 g) was added. The suspension was stirred at 25 °C for 1 hour. The solvent was then evaporated under a reduced pressure at 40 °C. The product was milled through a 0.50 mm sieve and then dried overnight in vacuum at 60 °C to give 473 g of the adsorbate.

### Example 8: Tadalafil - Aerosil^{®} 380 adsorbate

Tadalafil (2 g) was suspended in dichloromethane (300 ml). The mixture was heated to reflux and stirred at this temperature for 30 minutes. When all of the material dissolved, the clear solution was cooled to 25 °C and Aerosil^{®} 380 (10 g) was added. The suspension was stirred at 25 °C for 1 hour. The solvent was then evaporated under a reduced pressure at 40 °C. The product was milled through a 0.50 mm sieve and then dried overnight in vacuum at 60 °C to give 8 g of the adsorbate.

### Example 9: Tadalafil - Aerosil^{®} 380 adsorbate

Tadalafil (70 g) was suspended in dichloromethane (8.5 I). The mixture was heated to reflux and stirred at this temperature for 30 minutes. When all of the material dissolved, the clear solution was cooled to 25 °C and Aerosil^{®} 380 (350 g) was added. The suspension was stirred at 25 °C for 1 hour. The solvent was then evaporated under a reduced pressure at 40 °C. The product was milled through a 0.50 mm sieve and then dried overnight in vacuum at 60 °C to give 383 g of the adsorbate.

### Example 10: Tadalafil - Aerosil^{®} 380 adsorbate

Tadalafil (2.4 g) was suspended in dichloromethane (450 ml). The mixture was heated to reflux and stirred at this temperature for 30 minutes. When all of the material dissolved, a clear solution was cooled to 25 °C and Aerosil^{®} 380 (10 g) was added. The suspension was stirred at 25 °C for 1 hour using a rotor-stator mixer (Ultraturax) at 8000 rpm. The solvent was then evaporated under reduced pressure at 40 °C. The product was milled through 0.50 mm sieve and then dried overnight in vacuum at 60 °C to give 9.4 g of adsorbate.

### Example 11: Tadalafil - Aerosil^{®} 380 adsorbate

Tadalafil (2.2 g) was suspended in dichloromethane (450 ml). The mixture was heated to reflux and stirred at this temperature for 30 minutes. When all of the material dissolved, the clear solution was cooled to 25 °C and Aerosil^{®} 380 (10 g) was added. During stirring the suspension was subjected to ultrasound for 1 hour at 25 °C. The solvent was then evaporated under a reduced pressure at 40 °C. The product was milled through a 0.50 mm sieve and then dried overnight in vacuum at 60 °C to give 9.6 g of the adsorbate.

### Experiment 12: Tadalafil - Triethyl Citrate - Aerosil^{®} 380 adsorbate

Tadalafil (1.6 g) was suspended in dichloromethane (300 ml). The mixture was heated to reflux and stirred at this temperature for 30 minutes. When all of the material dissolved a clear solution was cooled to 25 °C and tryethyl citrate (0.48 g) was added. The mixture was stirred at 25 °C for 10 minutes. Aerosil^{®} 380 (10 g) was added. The suspension was stirred at 25 °C for 1 hour. The solvent was then evaporated under reduced pressure at 40 °C. The product was milled through 0.50 mm sieve and then dried overnight in vacuum at 60 °C to give 5 g of adsorbate.

## Claims

1. An adsorbate comprising tadalafil associated with a particulate and/or porous carrier.

2. The adsorbate according to claim 1 wherein tadalafil is in amorphous form.

3. The adsorbate according to claim 1 or 2, wherein tadalafil is essentially not in the form of crystals, and also preferably tadalafil is not deposited on the particulate carrier as a precipitate.

4. The adsorbate according to any one of the preceding claims, wherein the carrier is silicon dioxide, preferably colloidal silicon dioxide.

5. The adsorbate according to any of the preceding claims, wherein the amount of tadalafil in the adsorbate is in the range of 0.01 to 40 wt.-%.

6. The adsorbate according to any of the preceding claims, wherein the adsorbate further contains a substance selected from the group consisting of water soluble agents.

7. A dosage form comprising the adsorbate according to any of claims 1 - 6.

8. The dosage form according to any of the preceding claims, wherein the amount of the adsorbate in the pharmaceutical composition is in the range of 1 to 95 wt.-%, preferably in the range of 5 to 90 wt.-%.

9. A process for the preparation of an adsorbate comprising
a) providing an active pharmaceutical ingredient (API) being practically insoluble in water;
b) providing a solid support carrier in the form of particles;
c) placing said API and said carrier particles into a predetermined solvent which allows the adsorption of the API on the surface of carrier particles but gives substantially no API-precipitate, and
d) removing the solvent to form an API-carrier adsorbate.

10. The process according to claim 9, wherein the API is tadalafil and/or wherein the carrier is silicon dioxide, preferably colloidal silicon dioxide.

11. The process according to claims 9 or 10, wherein the solvent is a non-polar solvent.

12. The process according to any of claims 9 to 11, wherein at least one water soluble agent is added at any time during the process, preferably prior to step (d).

13. A process for the preparation of a dosage form comprising an adsorbate of an active pharmaceutical ingredient (API) being practically insoluble in water, associated with a particulate and/or porous carrier, the process comprising the steps of:
a) providing a mixture of the adsorbate, formed by the API being practically insoluble in water on the particulate carrier, and at least one excipient, preferably the excipient is a fine grade hydrophilic excipient,
b) fine-milling, preferably impact-milling of the mixture of step (a),
c) formulation of the mixture of step (b) into a dosage form by dry formulation technique.

14. Use of an adsorbate according to any of claims 1 to 6 for the preparation of a dosage form.

15. The dosage form according to claim 7 or 8 for use in the treatment of erectile dysfunction, hypercholesterolemia, human immunodeficiency virus (HIV) infections and/or Acquired Immune Deficiency Syndrome (AIDS).
